# EUROPEAN PATENT SPECIFICATION

(11) **EP 2 373 344 B1**
(45) Date of publication and mention of the grant of the patent: **30.03.2016**
(21) Application number: 09764230.0
(22) Date of filing: 27.11.2009
(51) Int. Cl.: A61K 47/20, A61K 31/41, A61K 31/4196, A61K 45/06

(54) **COMBINED DRUG ADMINISTRATION**
GEMEINSAME WIRKSTOFFVERABREICHUNG
ADMINISTRATION CONJOINTE

(30) Priority: 08.12.2008 EP 08170948
(43) Date of publication of application: 12.10.2011
(73) Proprietor: F. Hoffmann-La Roche AG, 4070 Basel (CH)
(72) Inventor: GROSS, Guenter, 79576 Weil am Rhein (DE); TARDIO, Joseph, F-68300 Saint Louis (FR)
(74) Representative: Salud, Carlos E.
(86) International application number: PCT/EP2009/065943
(87) International publication number: WO 2010/066593

(56) References cited:
- WO-A1-03/097714
- WO-A1-2004/082675
- WO-A2-03/000295
- WO-A2-2004/082593
- US-A1- 2004 248 934
- US-A1- 2006 153 913

## Description

The present invention is directed to the combined administration of an esterase inhibitor and a thioester.

In a first aspect the present invention provides a process for increasing the bioavailability of a thioester, wherein a dosage form containing an esterase inhibitor is administered in combination with a dosage form containing the thioester.

The term "esterase" denotes a group of enzymes which in a chemical reaction with water split esters into an acid and an alcohol, e.g. a thioester into an acid and a thioalcohol. Examples for an esterase inhibitor include inhibitors of human carboxylesterases (Satoh et al., Chemico-Biological Interactions 162:195-211 (2006) such as Benzil derivatives, e.g., 1-{4-[oxo(phenyl)acetyl]phenyl}-2-phenylethane-1,2-dione (Wadkins et al., J. Med. Chem. 48:2906-2915 (2005), or trifluoromethyl-ketones, e.g. 3-(dodecylsulfonyl)-1,1,1-trifluoropropane-2,2-diol (Wadkins et al., Molecular Pharmacology 71:713-723 (2007), or nitrophenylester derivatives, e.g. 4-benzyl-piperidine-1-carboxylic acid 4-nitrophenyl ester, or sulfonamide derivatives, e.g. 4-chloro-N-(4-{[(4-chlorophenyl)sulfonyl]amino}phenyl)benzenesulfonamide (Wadkins et al. Molecular Pharmacology 65(6):1336-1343 (2004). Other examples of esterase inhibitors include inhibitors of lipase such as orlistat and cetilistat (Birari and Bhutani, Drug Disc. Today 12:379-389 (2007)). Orlistat (tetrahydrolipstatin, 1-(3-hexyl-4-oxo-oxetan-2-yl)tridecan-2-yl 2-formyl-amino-4-methyl-pentanoate) is commercially available in a capsule dosage form. Each capsule contains the active ingredient, orlistat, and also contains the inactive ingredients microcrystalline cellulose, sodium starch glycolate, sodium lauryl sulfate, povidone, and talc.

The invention also provides a combination comprising a thioester, preferably of formula I, or prodrug that forms S-[2-([[1-(2-ethylbutyl) cyclohexyl] carbonyl] amino) phenyl] thiol *in vivo* and at least an esterase inhibitor.

In another embodiment the invention provides a pharmaceutical composition comprising (a) a thioester, preferably of formula (I) or a prodrug that forms S-[2-([[1-(2-ethylbutyl) cyclohexyl] carbonyl] amino) phenyl] thiol *in vivo,* (b) at least an esterase inhibitor and (c) one or more pharmaceutically acceptable carriers. The carriers or excipients must be acceptable in the sense of being compatibly with the other ingredients and not deleterious to the recipient thereof.

The invention further provides a package comprising separate dosage units, of which (a) at least one dosage unit comprises (a) a thioester, preferably of formula (I), or a prodrug that forms S-[2-([[1-(2-ethylbutyl) cyclohexyl] carbonyl] amino) phenyl] thiol *in vivo,* and (b) at least one other dosage unit comprises an esterase inhibitor. A"package"is understood to be any package useful for stable storage of the dosage units. The package may, for example, be a glass or plastic (e.g., a high-density polyethylene) container generally used for packaging and storage of tablets. Another form of packaging is a blister pack. Blister packs are well known in the packaging industry and are being widely used for the packaging of pharmaceutical unit dosage forms (e.g., tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil, at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via the opening.

The invention additionally provides a kit comprising (a) a first pharmaceutical composition comprising a therapeutically effective amount of (i) a thioester, preferably of formula (I), or a prodrug that forms S-[2-([[1-(2-ethylbutyl) cyclohexyl] carbonyl] amino) phenyl] thiol *in vivo,* and (ii) a pharmaceutically acceptable carrier, (b) a second pharmaceutical composition comprising (i) at least one esterase inhibitor, and (ii) a pharmaceutically acceptable carrier, (c) prescribing information, and (d) a container, wherein the first and second pharmaceutical compositions can be the same or different, and wherein the prescribing information includes advice to a patient regarding co-administration of a thioester, preferably of formula (I), or a prodrug that forms S-[2-([[1-(2-ethylbutyl) cyclohexyl] carbonyl] amino) phenyl] thiol *in vivo,* and the esterase inhibitor.

The invention additionally provides a method for the treatment or prophylaxis of a cardiovascular disorder in a patient, which comprises treating the patient with a therapeutically effective amount of a combination of (a) a thioester, preferably of formula (I), or a prodrug that forms S-[2-([[1-(2-ethylbutyl) cyclohexyl] carbonyl] amino) phenyl] thiol *in vivo,* and (b) at least one esterase inhibitor. Preferably the esterase inhibitor is administered prior to the administration of the dosage form containing the thioester.

The cardiovascular disorders include, but are not limited to, cardiovascular disease, coronary heart disease, coronary artery disease, hypoalphalipoproteinemia (low levels of HDL cholesterol), hyperbetalipoproteinemia (high levels of LDL cholesterol), hypercholesterolemia, hyperlipidemia, and atherosclerosis. Additional cardiovascular disorders which can be treated or prevented include, but are not limited to, hypertension, hypertriglyceridemia, hyperlipidoproteinemia, peripheral vascular disease, angina, ischemia, primary hypercholesterolemia (homozygous and heterozygous familial and nonfamilial), mixed dylipidemis (Frederickson Types IIa and IIb), and myocardial infarction. Following treatment with the above-described combination, the progression of atherosclerotic plaques is preferably slowed or arrested (e.g., in coronary arteries, in carotid arteries, and/or in the peripheral arterial system) in a patient. Preferably, the atherosclerotic plaques regress following treatment (e.g., in coronary arteries, in carotid arteries, and/or in the peripheral arterial system) in a patient.

Examples for a thioester include those disclosed e.g. in EP 1020439 A1, e.g. compounds of formula I 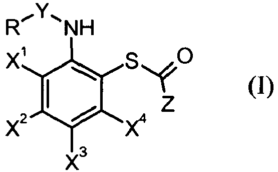 wherein
- R: is C₁-C₁₀alkyl, C₂-C₁₀alkenyl, haloC₁-C₄alkyl, C₃-C₁₀cycloalkyl, C₅-C₈cycloalkenyl, C₃-C₁₀cycloalkylC₁-C₁₀alkyl, aryl, aralkyl or a 5- or 6-membered heterocyclic group having 1 to 3 nitrogen, oxygen or sulfur atoms,
- X¹, X², X³ and X⁴: independently are hydrogen, halogen, C₁-C₄alkyl, haloC₁-C₄alkyl, C₁-C₄alkoxy, cyano, nitro, acyl or aryl,
- Y: is -CO- or -SO₂; and
- Z: is C₁-C₁₀alkyl, C₃-C₁₀cycloalkyl or C₃-C₁₀cycloalkylC₁-C₁₀alkyl.

In a preferred embodiment of the present invention the thioester is thioisobutyric acid S-(2-{[1-(2-ethyl-butyl)-cyclohexanecarbonyl]-amino}-phenyl) ester.

The thioester may be formulated in any conventional formulation including capsule formulations or tablet formulations. An example of a thioester tablet formulation is disclosed in WO2004/082593 and given in Table 1.

**Table 1: Example of a tablet formulation of COMPOUND A**

| Ingredient | amount (mg) | content (%) |
|---|---|---|
| Thioester (COMPOUND A) | 300 | 54.8 |
| Hydroxypropyl methylcellulose 2910 | 18 | 3.3 |
| Crospovidone | 119.8 | 21.9 |
| Talc | 18 | 3.3 |
| Low-substituted Hydroxypropyl cellulose | 90 | 16.5 |
| Magnesium stearate | 1.2 | 0.2 |
| Total | 547 | 100.0 |

The combined oral administration of an esterase inhibitor and a thioester provides for an increase in stability and the bioavailability of the thioester in the gastrointestinal tract of patients which is shown by the following test results:

### Example 1: Stability of thioisobutyric acid S-(2-{[1-(2-ethyl-butyl)-cyclohexanecarbonyl]-amino}-phenyl) ester

### Stock solutions:

(a) FeSSIF (Fed State Simulated Intestinal Fluid) and phosphate buffered saline solution:

| Ingredient | phosphate buffered saline solution | Pro-FeSSIF 1.11x | Concentrate (FeSSIF 10x) | FeSSIF |
|---|---|---|---|---|
| Sodium chloride | 3.2 g | 6.6 g | | 202 mM |
| Sodium dihydrogenphosphate dihydrate | 2.0 g | | | ----- |
| Acetic acid | | 4.8 g | | 144 mM |
| Sodium taurocholate | | | 806 mg | 15 mM |
| Lecithin | | | 300 mg | 3.75 mM |
| Sodium hydroxide 1N | ad pH 6.5 | ad pH 6.5 | | pH 6.5 |
| Water to total volume of | 500 mL | 500 mL | 10 mL | |

| | | | | |
|---|---|---|---|---|
| FeSSIF = 1 part concentrate + 9 parts Pro-FeSSIF | | | | |

(b) Stock A = drug solution (0.5 mg/mL):
5 mg ofthioisobutyric acid S-(2-{[1-(2-ethyl-butyl)-cyclohexanecarbonyl]-amino}-phenyl) ester (COMPOUND A) were dissolved in 50 µL ethanol and 950µL mixed micelles Soybean lecithin / Sodium glycocholate (158 / 97 mg/mL) in water were added. One part of the mixture was diluted with 9 parts of phosphate buffered saline solution at pH 6.5.

(c) Stock B = esterase inhibitor solution (1.0 mg/mL)
5 mg 1-(3-hexyl-4-oxo-oxetan-2-yl)tridecan-2-yl 2-formyl-amino-4-methyl-pentanoate (COMPOUND B) were dissolved in 250 µL ethanol. 4.75 mL mixed micelles soybean lecithin/sodium glycocholate (30/80.6 mg/mL) were added to give a solution of 1 mg/mL COMPOUND B.

(d) Stock C = Lipase in phosphate buffered saline solution (PBS) pH 6.5 (example given at 24 mg/mL, with a final enzyme activity of about 450 U/mL)
240 mg Lipase (Lipase from Hog Pancreas, 20.6U/mg Lipase, CAS No. 9001-62-1, Fluka, Art. No. 62300) were weighed into a vial and dissolved by stirring in 10 mL phosphate buffered saline solution pH 6.5. Any undissolved material was removed by centrifugation during 5 min at 5000rpm. The supernatant was used for the experiments and diluted with PBS to obtain the Lipase at the final concentration of 0.55 mg/mL.
200 µL 1-(3-hexyl-4-oxo-oxetan-2-yl)tridecan-2-yl 2-formyl-amino-4-methyl-pentanoate solution (stock B) were injected in 1.6 mL Lipase solution (stock C)(from stock C to obtain a final enzyme concentration of 0.55 mg/mL). To this, 200 µL drug solution (stock A) were injected at time zero. The mixture was incubated at 37°C, while mixing gently with a rotating shaker at about 2rpm. At regular intervals 200 µL samples were taken and diluted immediately with 600 µL isopropanol. The combined solution/suspension was centrifuged during 5 min at 3000 rpm and the concentration of COMPOUND A as well as its potential degradation products were determined in the supernatant by HPLC analysis.

To obtain the value at time zero, 600 µL isopropanol were added to 180 µL of a 0.55 mg/mL Lipase in phosphate buffered saline solution to inhibit the enzymes followed by the addition of 20 µL drug solution (stock A).

| **Time** | **+100µg/mL** COMPOUND B | | **w/o** COMPOUND B | |
|---|---|---|---|---|
| **(min)** | **COMPOUND A** | **Disulfide** | **COMPOUND A** | **Disulfide** |
| 0 | 46.9 | | | |
| 5 | 48.2 | < 0.2 | 27.2 | 16.4 |
| 10 | 48.2 | < 0.2 | 14.7 | 26.8 |
| 15 | 48.2 | < 0.2 | 7.3 | 33.1 |
| 20 | 48.0 | < 0.2 | 4.1 | 36.3 |
| 30 | 48.3 | < 0.2 | < 0.1 | 38.0 |
| 60 | 40.1 | < 0.2 | < 0.1 | 32.3 |

| | | | | |
|---|---|---|---|---|
| Concentration in µg/mL of COMPOUND A (theoretical concentration is 50 µg/mL) and Disulfide with 0.55 mg/mL (11U/mL) Lipase | | | | |

### Example 2: Comparison of different experimental procedures and influence of the lipase inhibitor concentration on stability of thioisobutyric acid S-(2-{[1-(2-ethylbutyl)-cyclohexanecarbonyl]-amino}-phenyl) ester

**Procedure A** (Premix of Lipase and COMPOUND B, followed by addition of COMPOUND A): 1600 µL Lipase in phosphate buffered saline solution (from stock C) to obtain a final enzyme concentration of 0.55 mg/mL) were mixed with 200 µL COMPOUND B (stock B) and preincubated during 5 min at 37°C. At time zero 200 µL drug solution (stock A) were added. The mixture was incubated at 37°C. After 10 min, the reaction was stopped by dilution of one part of the mixture with 3 parts of isopropanol.

**Procedure B** (Premix of COMPOUND B and COMPOUND A, followed by addition of Lipase): 100 µL drug solution (stock A) were mixed with 0, 12, 25 or 50 µL esterase inhibitor solution (stock B) and 100, 88, 75 or 50 µL FeSSIF concentrate (Lecithin37.5 mM and Sodium taurocholate 150 mM) were added to make up 200 µL of a solution with COMPOUND B concentrations of 0, 12, 25 or 50 µg/mL. At time zero, 800 µL Lipase in phosphate buffered saline solution (from stock C to obtain final enzyme concentrations of 0.55 mg/mL) were added. The reaction mixture was incubated at 37°C and stopped after 10 minutes by diluting one part of the reaction mixture with 3 parts Isopropanol.

| | Procedure A (Lipase + COMPOUND B, then COMPOUND A) | | Procedure B (COMPOUND B + COMPOUND A, then Lipase) | |
|---|---|---|---|---|
| COMPOUND B (µg/mL) | COMPOUND A (µg/mL) | SD (n =3) | COMPOUND A (µg/mL) | SD (n =3) |
| 0 | 14.7 | n.d. | 23.0 | 1.0 |
| 12 | n.d. | n.d. | 46.4 | 0.6 |
| 25 | n.d. | n.d. | 46.5 | 1.0 |
| 50 | 46.4 | 0.7 | 46.1 | 1.1 |
| 100 | 48.2 | 0.8 | n.d. | n.d. |

| | | | | |
|---|---|---|---|---|
| Concentration in µg/mL of COMPOUND A (theoretical concentration 50 µg/ml) with 0.55 mg/mL (11U/mL) Lipase | | | | |

**Procedure B'** (Premix of COMPOUND B and COMPOUND A, followed by addition of Lipase):
The tests were repeated as described under procedure B but with final COMPOUND B concentration of 0, 3, 6, 12 µg/ml.

| COMPOUND B (µg/ml) | COMPOUND A (µg/ml) |
|---|---|
| 0 | 14.2 |
| 3 | 45.9 |
| 6 | 46.5 |
| 12 | 45.7 |

| | |
|---|---|
| Concentration in µg/mL of COMPOUND A (theoretical concentration is 50 µg/mL) after 10 min incubation with 0.55 mg/mL (11U/mL) Lipase | |

**Procedure C** (Premix of COMPOUND A and Lipase, followed by addition of COMPOUND B):
Remark: Final COMPOUND B concentration: 3 µg/ml
1^{st} experiment:
   220 µL drug solution (stock A) and 220 µL FeSSIF concentrate (Lecithin37.5 mM and Sodium taurocholate 150 mM) were mixed. At time zero the reaction was started by addition of 1700 µl Lipase in phosphate buffered saline solution (from stock C to obtain a final enzyme concentration of 0.55 mg/mL). The mixture was incubated at 37°C and after 5 min, a 200 µL sample was taken for HPLC analysis. Then 60 µL COMPOUND B solution (stock B) previously diluted 1:10 in phosphate buffered saline solution (corresponds to 100 µg/mL COMPOUND B) was added and as a function of time, samples of 200 µL were taken, diluted immediately with 600 µL isopropanol and centrifuged for 5 min at 3000 rpm. The supernatant was analyzed by HPLC.
2^{nd} experiment (identical to experiment 1 but different order of reagent addition):
   1700 µl Lipase in phosphate buffered saline solution (from stock C to obtain a final enzyme concentration of 0.55 mg/mL) were mixed with 220 µL FeSSIF concentrate and at time zero 220 µL drug solution (stock A) was added. After 7 min of incubation, a 200 µL sample was taken, 60 µL diluted COMPOUND B solution (stock B) was added and continued as described for the 1^{st} experiment.

| Time | +3µg/mL COMPOUND B at T= 5min | | +3µg/mL COMPOUND B at T= 7min | |
|---|---|---|---|---|
| (min) | COMPOUND A | Disulfide | COMPOUND A | Disulfide |
| 0 | 45.3 | | 45.3 | |
| 5 | 41.5 | 5.3 | 40.4 | 6.6 |
| 10 | 39.1 | 5.8 | 38.4 | 7.6 |
| 15 | 39.3 | 5.6 | 38.2 | 7.2 |
| 20 | 39.1 | 5.6 | 37.9 | 7.6 |
| 25 | 39.3 | 5.8 | 37.8 | 7.6 |
| 30 | 39.3 | 5.8 | 37.9 | 7.2 |

| | | | | |
|---|---|---|---|---|
| Concentration in µg/mL of COMPUND A (theoretical concentration is 50 µg/mL) and Disulfide after incubation with 0.55 mg/mL (11U/mL) Lipase | | | | |

By these experiments it could be demonstrated that the lipase inhibitor COMPOUND B efficiently prevents lipase induced hydrolytic degradation of COMPOUND A. The presence of the esterase inhibitor effectively maintained the stability of COMPOUND A and inhibited formation of disulfide.

### Example 3: Influence of the lipase inhibitor concentration on stability of thioisobutyric acid S-(2-{[1-(2-ethyl-butyl)-cyclohexanecarbonyl]-amino}-phenyl) ester in human intestinal fluids

**Procedure D** (COMPOUND A and COMPOUND B in solution):
**Fasted Intestinal Fluids:** the fluids from 4 different subjects were collected with a nasojejunal tube after fasting overnight and pooled.
**Stimulated Intestinal Fluids:** the fluids from 3 different subjects were collected after fasting overnight with a nasojejunal tube and pooled. For the stimulation, twenty minutes before the beginning of the collection, volunteers were provided with chewing-gums and were asked to chew over a 15 minutes period, maintaining the taste by replacing the sticks of gum approximately every 2 minutes.

### Stock solutions

**(a) Stock D = drug** solution (5 mg/mL):
   5 mg of thio isobutyric acid S-(2-{[1-(2-ethyl-butyl)-cyclohexanecarbonyl]-amino}-phenyl) ester (COMPOUND A) were dissolved in 50 µL ethanol and 950µL mixed micelles solution containing Soybean lecithin / Sodium glycocholate (158 / 97 mg/mL) in water were added.
**(b) Stock E =** esterase inhibitor solution (0.3 mg/mL)
   3 mg 1-(3-hexyl-4-oxo-oxetan-2-yl)tridecan-2-yl 2-formyl-amino-4-methyl-pentanoate (COMPOUND B) were dissolved in 1 mL ethanol. 100 µL of this solution was diluted with 900 µL FeSSIF concentrate (10x).
(c) **Stock F** = premix of COMPOUND A and COMPOUND B (respectively 500 and 30 µg/mL) 100 µL drug solution (stock D) were mixed with 100 µL esterase inhibitor solution (stock E) and 800 µL pro-FaSSIF were added.
(d) **Stock G =** premix of COMPOUND A and COMPOUND B (respectively 500 and 10 µg/mL) 100 µL premix (stock F) were mixed with 200 µL drug solution (stock A).
(e) **Stock H =** premix of COMPOUND A and COMPOUND B (respectively 500 and 20 µg/mL) 200 µL premix (stock F) were mixed with 100 µL drug solution (stock A).
315 µL intestinal fluids (fasted and stimulated) were pre-heated at 37°C for about 5 min. At time zero, 35 µL of premix solutions were added, i.e. either stock A, stock G, stock H or stock F, to obtain 50 µg/mL of COMPOUND A and final concentrations of COMPOUND B of 0, 1, 2 or 3 µg/mL. The samples were incubated at 37°C while gently stirring. The reaction was stopped after 2 min or after 10 min by dilution of 1 part of the reaction mixture with 3 parts of isopropanol and the resulting mixture was centrifuged for 5 min at 5'000 rpm. The supernatant was analyzed by HPLC.

| COMPOUND B conc. (µg/mL) | COMPOUND A (µg/mL) | | Disulfide (µg/mL) | |
|---|---|---|---|---|
| | 2 min | 10 min | 2 min | 10 min |
| 0 | 0.48 | 0.48 | 31.56 | 32.68 |
| 1 | 18.00 | 1.58 | 15.43 | 21.53 |
| 2 | 36.91 | 28.59 | 7.75 | 10.66 |
| 3 | 43.72 | 37.24 | 5.16 | 6.81 |

| | | | | |
|---|---|---|---|---|
| Concentration in µg/mL of COMPOUND A (theoretical concentration is 50 µg/mL) and Disulfide in intestinal fluid obtained from fasted subjects | | | | |

| COMPOUND B conc. (µg/mL) | COMPOUND A (µg/mL) | | Disulfide (µg/mL) | |
|---|---|---|---|---|
| | 2 min | 10 min | 2 min | 10 min |
| 0 | 0.48 | 0.48 | 39.95 | 40.16 |
| 1 | 26.06 | 2.76 | 18.82 | 28.59 |
| 2 | 38.92 | 24.93 | 10.79 | 14.95 |
| 3 | 46.91 | 40.48 | 8.33 | 10.36 |

| | | | | |
|---|---|---|---|---|
| Concentration in µg/mL of COMPOUND A (theoretical concentration is 50 µg/mL) and Disulfide in intestinal fluid obtained from subjects after stimulation | | | | |

By the above experiments it is evident that the presence of COMPOUND B stabilizes dissolved COMPOUND A towards hydrolysis in intestinal fluids and reduces the formation of the disulfide.

### Example 4: Influence of the lipase inhibitor concentration on stability of thioisobutyric acid S-(2-{[1-(2-ethyl-butyl)-cyclohexanecarbonyl]-amino}-phenyl) ester in human intestinal fluids

**Procedure E** (COMPOUND A and COMPOUND B in suspension, excess compound separated by centrifugation):
**Stock J** = COMPOUND B suspension (2 mg/mL)
   100 µL FeSSIF concentrate and 900 µL pro-FeSSIF pH6.5 were added to 4 mg commercially available Orlistat formulation (Xenical^{®}) containing 2 mg lipase inhibitor 1-(3-hexyl-4-oxo-oxetan-2-yl)tridecan-2-yl 2-formyl-amino-4-methyl-pentanoate (COMPOUND B) in a vial and the formulation was dispersed by gently stirring during 15 min to obtain a fine homogenous suspension.

500 µL freshly thawed intestinal fluids (fasted and stimulated state) were added to 1 mg COMPOUND A in a 1 mL glass vial immediately followed by the addition of either 0, 10 or 20 µL COMPOUND B suspension (stock J) corresponding to 0, 40 or 80 µg/mL COMPOUND B. The amount of COMPOUND B was 0, 2% or 4% COMPOUND B relative to COMPOUND A. The mixture was incubated at 37°C with gentle stirring. Samples of 90 µL were removed as function of time and centrifuged at 13'000 rpm (about 12'000 xg) for 5 min. 50 µL of the supernatant was diluted with 150 µL isopropanol, centrifuged at 5'000 rpm for 5 min and the clear supernatant was analyzed by HPLC.

| | HIF-fasted (µg/mL) | | | HIF-stimulated (µg/mL) | | |
|---|---|---|---|---|---|---|
| Time | COMPOUND A | Thiophenol | Disulfide | COMPOUND A | Thiophenol | Disulfide |
| 15 min | 1.62 | 0.00 | 218.12 | 3.09 | 2.55 | 49.80 |
| 30 min | 1.86 | 3.41 | 514.94 | 0.52 | 24.33 | 158.53 |
| 1h | 0.49 | 29.55 | 994.29 | 0.62 | 89.14 | 348.73 |
| 2h | 0.49 | 125.39 | 1260.90 | 0.82 | 149.88 | 500.57 |
| 4h | 0.49 | 151.84 | 1313.31 | 1.31 | 208.98 | 619.27 |

| | | | | | | |
|---|---|---|---|---|---|---|
| Concentration in µg/mL of COMPOUND A (Thioester), Thiophenol and Disulfide in fasted and stimulated human intestinal fluids (HIF) without COMPOUND B. | | | | | | |

| Time | HIF-fasted (µg/mL) | | | HIF-stimulated (µg/mL) | | |
|---|---|---|---|---|---|---|
| | COMPOUND A | Thiophenol | Disulfide | COMPOUND A | Thiophenol | Disulfide |
| 15 min | 1.12 | 0.16 | 110.57 | 0.74 | 0.16 | 40.71 |
| 30 min | 11.75 | 3.85 | 363.56 | 0.55 | 0.89 | 102.65 |
| 1h | 111.05 | 13.54 | 646.50 | 10.53 | 75.39 | 343.60 |
| 2h | 217.35 | 25.60 | 902.02 | 32.71 | 149.23 | 565.70 |
| 4h | 281.66 | 64.21 | 1075.49 | 221.31 | 349.31 | 764.51 |

| | | | | | | |
|---|---|---|---|---|---|---|
| Concentration in µg/mL of COMPOUND A (Thioester), Thiophenol and Disulfide in fasted and stimulated human intestinal fluids (HIF) with 40 µg/mL COMPOUND B. | | | | | | |

| Time | HIF-fasted (µg/mL) | | | HIF-stimulated (µg/mL) | | |
|---|---|---|---|---|---|---|
| | COMPOUND A | Thiophenol | Disulfide | COMPOUND A | Thiophenol | Disulfide |
| 15 min | 5.62 | 0.16 | 31.75 | 1.00 | 0.48 | 54.34 |
| 30 min | 16.36 | 0.16 | 95.68 | 11.80 | 4.51 | 115.01 |
| 1h | 89.60 | 2.11 | 281.19 | 64.63 | 6.38 | 211.64 |
| 2h | 230.34 | 8.13 | 458.77 | 131.64 | 7.64 | 291.49 |
| 4h | 336.00 | 12.61 | 578.53 | 171.25 | 7.11 | 345.98 |

| | | | | | | |
|---|---|---|---|---|---|---|
| Concentration in µg/mL of COMPOUND A (Thioester), Thiophenol and Disulfide in fasted and stimulated human intestinal fluids (HIF) with 80 µg/mL COMPOUND B. | | | | | | |

The above experiments show that the addition of COMPOUND B in form of a suspension stabilizes the thioester which is present in excess in intestinal fluids against hydrolysis and subsequent oxidation of the thiol formed.

### Example 5: Influence of the lipase inhibitor concentration on stability of thioisobutyric acid S-(2-{[1-(2-ethyl-butyl)-cyclohexanecarbonyl]-amino}-phenyl) ester in human intestinal fluids

Procedure F (COMPOUND A and COMPOUND B in suspension, the degradation immediately stopped without separation of excess compound):
Stock K = COMPOUND B suspension (0.5 mg/mL)
   Dilute 1 part of COMPOUND B suspension (stock J) with 3 parts of FeSSIF pH6.5 solution.

500 µL freshly thawed intestinal fluids (fasted and stimulated state) were added to 1.2 mg COMPOUND A in a 1 mL glass vial. The suspended compound was stirred for 15 min at 37°C to obtain wetting and a homogenous suspension. In parallel, 4 glass vials were prepared with 20 µL of a COMPOUND B suspension at different concentrations. These concentrations were obtained by mixing FeSSIF pH6.5 and COMPOUND B suspension (stock K) in different ratios, respectively 1:0, 1:3, 1:1, 0:1 to obtain 0, 1, 2 and 4% related to COMPOUND A. At time zero, 100 µL COMPOUND A suspension preincubated at 37°C were added to the 20 µL COMPOUND B suspension. The mixture was incubated at 37°C with gentle stirring. After 15 min and 60 min, the reaction was stopped by mixing a 50 µL sample with 150 µL isopropanol and centrifuged at 5'000 rpm during 5 min. The clear supernatant was analyzed for the hydrolysis product and its oxidized form by HPLC.

| | T = 15 min | | | T = 60 min | | |
|---|---|---|---|---|---|---|
| COMPOUND B conc. (µg/mL) | COMPOUND A (µg/mL) | Thiophenol (µg/mL) | Disulfide (µg/mL) | COMPOUND A (µg/mL) | Thiophenol (µg/mL) | Disulfide (µg/mL) |
| 0 | 1111.92 | 12.07 | 512.32 | 182.50 | 22.18 | 1004.67 |
| 20 | 1299.01 | 9.58 | 367.37 | 718.26 | 15.27 | 569.98 |
| 40 | 1381.23 | 5.42 | 290.53 | 598.81 | 8.36 | 331.72 |
| 80 | 1728.95 | 6.34 | 290.06 | 826.93 | 10.49 | 299.09 |

| | | | | | | |
|---|---|---|---|---|---|---|
| Concentration in µg/mL of Compound A, Thiophenol and Disulfide in fasted human intestinal fluids (HIF) with 0, 20, 40 and 80 µg/mL COMPOUND B corresponding to 0, 1, 2 and 4% with regard to the Thioester concentration. | | | | | | |

| | T = 15 min | | | T = 60 min | | |
|---|---|---|---|---|---|---|
| COMPOUND B conc. (µg/mL) | COMPOUND A (µg/mL) | Thiophenol (µg/mL) | Disulfide (µg/mL) | COMPOUND A (µg/mL) | Thiophenol (µg/mL) | Disulfide (µg/mL) |
| 0 | 1569.58 | 68.88 | 392.71 | 400.63 | 412.83 | 903.76 |
| 20 | 2034.38 | 13.26 | 252.67 | 1503.21 | 29.88 | 459.09 |
| 40 | 2163.01 | 8.30 | 215.76 | 1515.41 | 20.48 | 269.78 |
| 80 | 1945.50 | 5.58 | 186.77 | 1449.03 | 8.51 | 212.28 |

| | | | | | | |
|---|---|---|---|---|---|---|
| Concentration in µg/mL of Compound A, Thiophenol and Disulfide in fasted human intestinal fluids (HIF) with 0, 20, 40 and 80 µg/mL COMPOUND B corresponding to 0, 1, 2 and 4% with regard to the Thioester concentration. | | | | | | |

By these experiments it could be demonstrated that the esterase inhibitor COMPOUND B efficiently prevents gastrointestinal enzyme induced hydrolytic degradation of COMPOUND A.

## Claims

1. A combination comprising a thioester and at least an esterase inhibitor.

2. A pharmaceutical composition comprising (a) a thioester , (b) at least an esterase inhibitor and (c) one or more pharmaceutically acceptable carriers.

3. A package comprising separate dosage units, of which at least one dosage unit comprises (a) a thioester, and (b) at least one other dosage unit comprises an esterase inhibitor.

4. A kit comprising (a) a first pharmaceutical composition comprising a therapeutically effective amount of (i) a thioester, and (ii) a pharmaceutically acceptable carrier, (b) a second pharmaceutical composition comprising (i) at least one esterase inhibitor, and (ii) a pharmaceutically acceptable carrier, (c) prescribing information, and (d) a container, wherein the first and second pharmaceutical compositions can be the same or different, and wherein the prescribing information includes advice to a patient regarding co-administration of a thioester and the esterase inhibitor.

5. A combination, a pharmaceutical composition, a package or a kit according to claim 1, 2, 3 or 4 respectively, wherein the thioester is of formula (I) 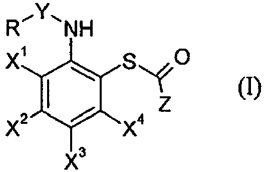 wherein
R is C₁-C₁₀alkyl, C₂-C₁₀alkenyl, haloC₁-C₄alkyl, C₃-C₁₀cycloalkyl, C₅-C₈cycloalkenyl, C₃-C₁₀cycloalkylC₁-C₁₀alkyl, aryl, aralkyl or a 5- or 6-membered heterocyclic group having 1 to 3 nitrogen, oxygen or sulfur atoms,
X¹, X², X³ and X⁴ independently are hydrogen, halogen, C₁-C₄alkyl, haloC₁-C₄alkyl, C₁-C₄alkoxy, cyano, nitro, acyl or aryl,
Y is-CO- or -SO₂; and
Z is C₁-C₁₀alkyl, C₃-C₁₀cycloalkyl or C₃-C₁₀cycloalkylC₁-C₁₀alkyl.

6. A combination, a pharmaceutical composition, a package or a kit according to claim 5, wherein the esterase inhibitor is 1-(3-hexyl-4-oxo-oxetan-2-yl)tridecan-2-yl 2-formyl-amino-4-methyl-pentanoate.

7. A combination, a pharmaceutical composition, a package, a kit according to claim 1, 2, 3, 4, 5 or 6 wherein the thioester is thioisobutyric acid S-(2-{[1-(2-ethyl-butyl)-cyclohexanecarbonyl]-amino}-phenyl) ester.

## Patentansprüche

1. Eine Kombination, umfassend einen Thioester und mindestens einen Esteraseinhibitor.

2. Ein Arzneimittel, umfassend (a) einen Thioester, (b) mindestens einen Esteraseinhibitor und (c) einen oder mehrere pharmazeutisch verträgliche Träger.

3. Eine Packung, umfassend separate Dosiereinhciten, von denen mindestens eine Dosiereinheit (a) einen Thioester umfasst und (b) mindestens eine andere Dosiereinheit einen Esteraseinhibitor umfasst.

4. Ein Kit, umfassend (a) ein erstes Arzneimittel, umfassend eine therapeutisch wirksame Menge (i) eines Thioesters und (ii) einen pharmazeutisch verträglichen Träger, (b) ein zweites Arzneimittel, umfassend (i) mindestens einen Esteraseinhibitor und (ii) einen pharmazeutisch verträglichen Träger, (c) Verschreibungsinformation und (d) einen Behälter, wobei das erste und zweite Arzneimittel gleich oder verschieden sein können und wobei die Verschreibungsinformation Anweisung an einen Patienten bezüglich Coadministration eines Thioesters und des Esteraseinhibitors beinhaltet.

5. Eine Kombination, ein Arzneimittel, eine Packung oder ein Kit nach Anspruch 1, 2, 3
beziehungsweise 4, wobei der Thioester der Formel (I) 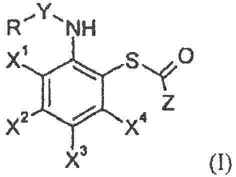 entspricht, wobei
R gleich C₁-C₁₀-Alkyl, C₂-C₁₀-Alkenyl, Halogen-C₁-C₄-alkyl, C₃-C₁₀-Cycloalkyl, C₅-C₈-Cycloalkenyl, C₃-C₁₀-Cycloalkyl-C₁-C₁₀-alkyl, Aryl, Aralkyl oder eine 5-odcr 6-gliedrige heterocyclische Gruppe, die 1 bis 3 Stickstoff-, Sauerstoff- oder Schwefelatome aufweist, ist,
X¹, X², X³ und X⁴ unabhängig Wasserstoff, Halogen, C₁-C₄-Alkyl, Halogen-C₁-C₄-alkyl, C₁-C₄-Alkoxy, Cyano, Nitro, Acyl oder Aryl sind,
Y gleich -CO- oder -SO₂ ist; und
Z gleich C₁-C₁₀-Alkyl, C₃-C₁₀-Cycloalkyl oder C₃-C₁₀-Cycloalkyl-C₁-C₁₀-alkyl ist.

6. Eine Kombination, ein Arzneimittel, eine Packung oder ein Kit nach Anspruch 5, wobei der Esteraseinhibitor 1-(3-Hexyl-4-oxo-oxetan-2-yl)tridecan-2-yl-2-formyl-amino-4-methyl-pentanoat ist.

7. Eine Kombination, ein Arzneimittel, eine Packung, ein Kit nach Anspruch 1, 2, 3, 4, 5 oder 6, wobei der Thioester Thioisobuttersäure-S-(2-{[1-(2-ethyl-butyl)-cyclohexancarbonyl]-amino}-phenyl)ester ist.

## Revendications

1. Association comprenant un thioester et au moins un inhibiteur d'estérase.

2. Composition pharmaceutique comprenant (a) un thioester, (b) au moins un inhibiteur d'estérase et (c) un ou plusieurs supports pharmaceutiquement acceptables.

3. Emballage comprenant des unités posologiques séparées, au moins une de ces unités posologiques comprenant (a) un thioester, et (b) au moins une autre unité posologique comprenant un inhibiteur d'estérase.

4. Kit comprenant (a) une première composition pharmaceutique comprenant une quantité thérapeutiquement efficace (i) d'un thioester, et (ii) un support pharmaceutiquement acceptable, (b) une seconde composition pharmaceutique comprenant (i) au moins un inhibiteur d'estérase, et (ii) un support pharmaceutiquement acceptable, (c) des informations de prescription et (d) un récipient, dans lequel les première et seconde compositions pharmaceutiques peuvent être identiques ou différentes, et dans lequel les informations de prescription comprennent l'information à un patient de la co-administration d'un thioester et de l'inhibiteur d'estérase.

5. Association, composition pharmaceutique, emballage ou kit suivant la revendication 1, 2, 3 ou 4, respectivement, dans lequel le thioester répond à la formule (I) 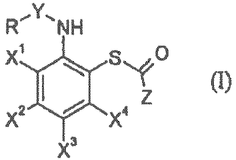 dans laquelle :
R représente un groupe alkyle en C₁ à C₁₀, alcényle en C₂ à C₁₀, halogénoalkyle en C₁ à C₄, cycloalkyle en C₃ à C₁₀, cycloalcényle en C₅ à C₈, (cycloalkyle en C₃ à C₁₀) (alkyle en C₁ à C₁₀), aryle, aralkyle, ou un groupe hétérocyclique penta- ou hexagonal ayant 1 à 3 atomes d'azote, d'oxygène ou de soufre,
X¹, X², X³ et X⁴ représentent indépendamment un atome d'hydrogène, un groupe halogéno, alkyle en C₁ à C₄, halogénoalkyle en C₁ à C₄, alkoxy en C₁ à C₄, cyano, nitro, acyle ou aryle,
Y représente un groupe -CO- ou -SO₂ ; et
Z représente un groupe alkyle en C₁ à C₁₀, cycloalkyle en C₃ à C₁₀ ou (cycloalkyle en C₃ à C₁₀) (alkyle en C₁ à C₁₀).

6. Association, composition pharmaceutique, emballage ou kit suivant la revendication 5, l'inhibiteur d'estérase étant le 2-formyl-amino-4-méthyl-pentanoate de 1-(3-hexyl-4-oxo-oxétane-2-yl)tridécane-2-yle.

7. Association, composition pharmaceutique, emballage ou kit suivant la revendication 1, 2, 3, 4, 5 ou 6, le thioester étant l'ester S-(2-{[1-(2-éthyl-butyl)-cyclohexane-carbonyl]-amino}-phénylique d'acide thioisobutyrique.
